Europäisches Patentamt

European Patent Office

Office européen des brevets

(11) Publication number: **0 076 080**
A2

# (12) EUROPEAN PATENT APPLICATION

(21) Application number: 82304957.2

(22) Date of filing: 21.09.82

(51) Int. Cl.³: **F 28 D 15/00**
A 61 F 7/00
//A42C5/04

(30) Priority: 25.09.81 GB 8129026

(43) Date of publication of application:
06.04.83 Bulletin 83/14

(84) Designated Contracting States:
DE FR SE

(71) Applicant: The Secretary of State for Defence in Her
Britannic Majesty's Government of The United Kingdom
of Great Britain and
Northern Ireland Whitehall
London SW1A 2HB(GB)

(72) Inventor: Graveney, Michael James
195 Frimley Green Road
Frimley Green Surrey(GB)

(74) Representative: Miller, Ronald Anthony et al,
Procurement Executive, Ministry of Defence Patents
1A4, Room 2014 Empress State Building Lillie Road
London SW6 1TR(GB)

(54) Improvements in or relating to heat pipes.

(57) Apparatus for cooling a scalp during administration of
drugs liable to cause Alopecia, the apparatus comprising a
heat pipe in conformable sheet form.

Fig.3.

EP 0 076 080 A2

IMPROVEMENTS IN HEAT PIPES

The present invention relates to heat pipes, especially those constituted as thermal conditioning garment assemblies for personnel use. It is particularly concerned with the provision of cooling to a person or animal undergoing medical or surgical treatment.

UK Patent Specification 1462033 describes a suit for substantially enveloping a human body, the surface of which for contacting the body substantially comprises a part of the outer surface of a ducting for a liquid, and which is constructed to allow in use access to parts of the torso for surgical operation, the suit comprising a bonnet member, arm members, a torso member, and a legs member, the members being preferably separate, and constructed as a labyrinthine duct formed by two sheets of plastics material.

UK Patent Specification 8106782 describes a flexible or conformable heat pipe assembly in sheet form and suitable for use as a thermal conditioning garment, the assembly having a reticulated structure including wicking and void continua, an impermeable plastics film envelope surrounding the structure, and means by which the assembly may be outgassed and evacuated and liquid introduced thereinto.

Certain cytotoxic (chemotherapeutic) drugs employed in the treatment of cancer have a propensity for being taken up into the hair roots of the patient, leading to temporary alopecia (loss of scalp hair). It has however been discovered that if the scalp

- 2

temperature can be arranged to be about $4^{\circ}$ centrigade for about 20 minutes before, during and after administration of cytoloxic drugs, alopecia can be prevented.

The present invention provides apparatus and process cooling a scalp.

According to a first aspect of the present invention apparatus for cooling a scalp comprises a heat pipe having

a conditioning heat exchanger portion constructed for embracing a scalp,

a reservoir heat exchanger portion, and

a communicating duct portion connecting the conditioning and the reservoir heat exchanger portions.

According to an important feature of the invention the apparatus may be formed integrally of heat pipe in flexible sheet form, the heat pipe having a reticulated structure including wicking and void continua, an impermeable plastics film envelope surrounding the structure, and means by which the heat pipe may be outgassed and evacuated and liquid introduced thereinto. Thus the conditioning heat exchanger portion may be constructed with a deeply dentate edge so that it can be formed to embrace a scalp of any shape, while the reservoir heat exchanger may be constructed with foldable flaps so that it can be formed into a container or a container lining.

The apparatus may include a scalp cap and ties for maintaining the conditioner portion in contact with the scalp. A particularly suitable scalp cap may comprise a bag of pellets, the bag fabric being substantially impermeable and the interior connected to evacuating means, whereby the bag can be formed over the conditioner portion on a user's head, and evacuated to retain the conditioner portion in close contact with the head. The communicating duct portion may be sheathed in thermally insulative material, and the heat exchanger portions backed by such material.

3

If the reservoir heat exchanger is in or in the form of a container it can contain ice or dry ice inter alia sufficient to provide cooling for the limited period required. Alternatively, the reservoir heat exchanger pipe may be connected to an electrical heat sink such as a thermoelectric device, refrigeration means, or heat pump. These are described in copending UK Patent application 8129051, 8129023, 8129052. Others are described in copending UK Patent applications 8129022, 8129050.

The flexible reticulated structure may be as described in UK Patent Specification 8106787, that is one formed in a three dimensional weaving process involving picking yarns out and thermosetting plastics warp and weft fibre elements, with the wicking perhaps constituting part or all of the weft or warp fibre or perhaps comprising a non woven sheet or sheets attached to or interwoven by the structure.

Whether the wicking forms part of the matrix or is introduced thereinto subsequent to formation thereof a suitable wicking may comprise glass fibre tow or cord, eg 'REFRASIL' (TM).

An alternative flexible reticulated structure may include an uncut velvet structure such as described in UK Patent Specification 1028141. Typically this could be formed of nylon, polyester, or carbon or glass fibre, with or without a stiffening agent.

The envelope suitably comprises a heat sealable plastics film, and is preferably metallised, eg aluminised. It may be a laminate of various materials, eg Mylar (RTM).

The heat pipe may have an associated pump, such as a roughing pump, for maintaining the required pressure in the heat pipe. Alternatively, where a scalp cap requiring an evacuating pump is employed, the pump may be arranged to serve both requirements.

4

A suitable method of fabricating a heat pipe according to the invention is described in UK Patent Specification 8106782 and comprises forming a flexible reticulated structure including a wicking element, forming an impervious flexible envelope to the structure and fitting a valve thereto, evacuating the interior of the envelope and introducing a quantity of a working liquid thereinto. After the introduction of the working liquid the heat pipe may be further out-gassed. A suitable working liquid, one which can be made to boil at the operating temperature by the application of about 50% vacuum in the apparatus, and which is preferably cheap and non toxic. Examples are methanol or one of the freon refrigerants. An inert gas may be added with the working liquid. Typically the heat pipe is pre-evacuated to $10^{-4}$ - $10^{-2}$ Torr in a manner which will substantially remove any non-condensable gases within the heat pipe.

A process of treatment of a hirsute skin according to another aspect of the present invention comprises covering the said skin with the conditioning portion of a heat pipe, and placing the reservoir portion of the heat pipe in contact with a low temperature source, the heat pipe being in accordance with the first aspect of the present invention, charged with a non-aqueous working liquid which has been outgassed by evacuation to $10^{-4}$ - $10^{-2}$ Torr.

Apparatus and methods of making and using it will now be described by way of example with reference to the accompanying drawing, of which:

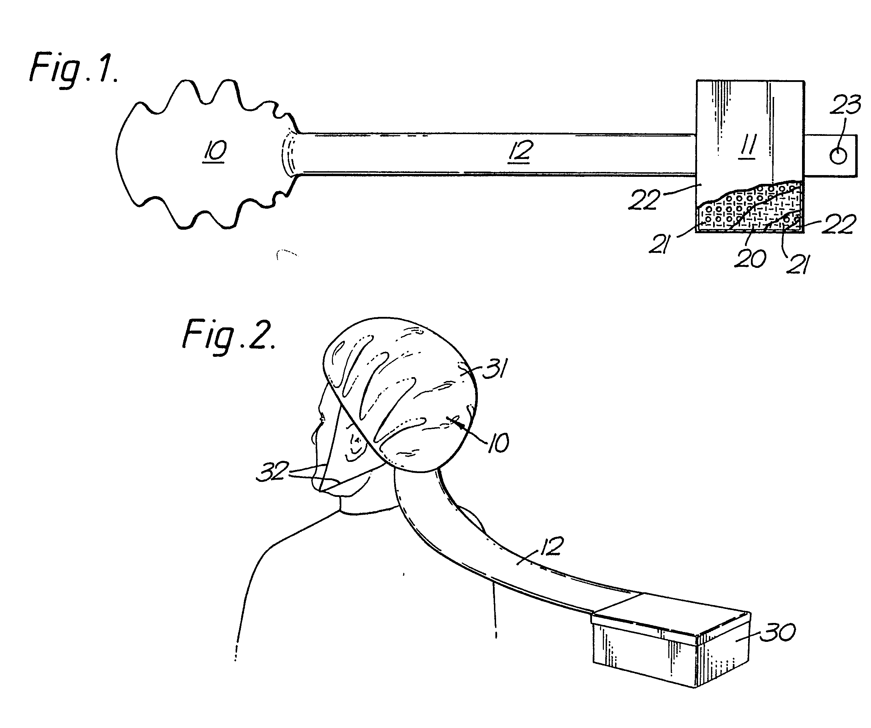

Figure 1 is a developed, part sectional view of a scalp cooling heat pipe,

Figure 2 is a view of the heat pipe in use,

Figure 3 is a section of a bead bag heat pipe skull cap.

The heat pipe shown in the drawings comprises a conditioning heat exchanger portion 10, a reservoir heat exchanging portion 11, and a communicating duct portion 12 xonnecting the two heat exchanging portions. It is integrally constructed, with a flexible reticulated structure 20 in sheet form having on both faces a perforated sheet 21 of wick material and enveloped in metallised plastics film 22. It has an outgassing and charging valve 23.

As can be seen in the drawings, by being deeply dentate the conditioning heat exchanger portion is conformable to the surface of a user's head, while the reservoir portion 11 is conformable to line a container 30. A cap 31 with ties 32 is employed to retain the portion 10 to a user's head.

To prepare the heat pipe for use it is evacuated at 23 and an appropriate quantity of ethanol is allowed to be drawn back into it. It is then again evacuated and outgassed. The portion 10 is formed to a user's head and restrained in position with the cap 31. The portion 11 is formed into a container liner, placed in a container and filled with solid carbon dioxide, and loosely covered.

Drugs liable to cause alopecia can then be administered to the person, and the heat pipe removed 10-30 minutes after the administration, depending on the method thereof.

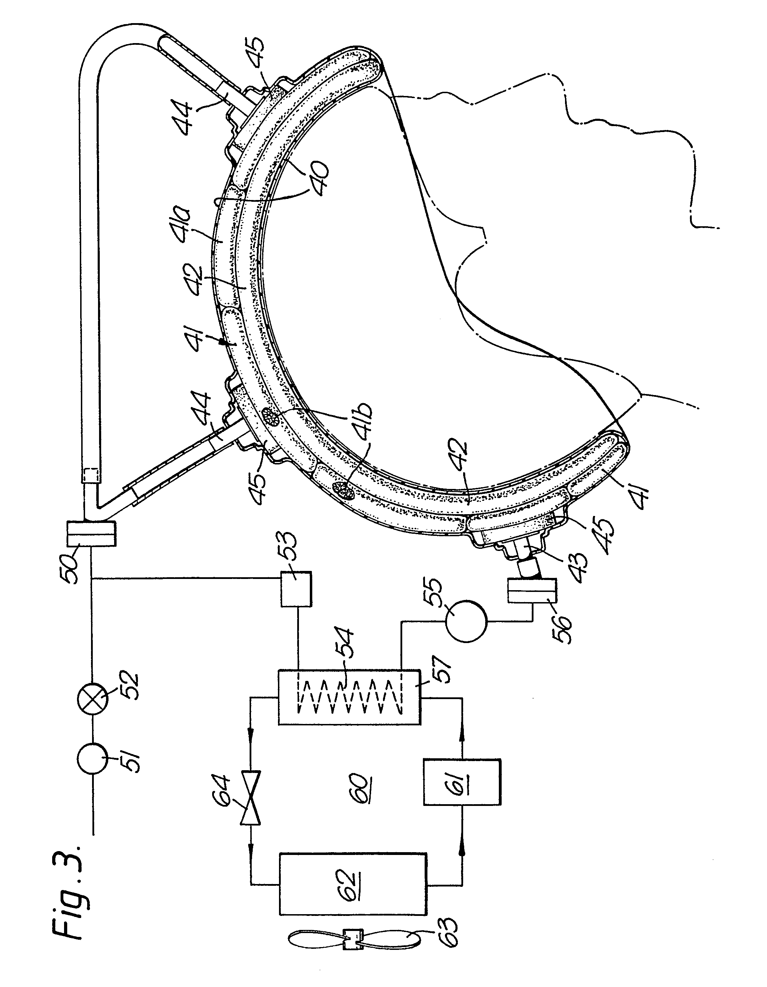

The bead bag heat pipe skull cap illustrated in Figure 3 comprises an impermeable sac 40 containing a plurality of bead bags 41 and a wick mat 42. The sac 40 has a liquid inlet part 43 and vacuum and vapour outlet ports 44, each port being faced with a 3-dimensionally reticulated diffuser 45. The bead bags 41 comprise a permeable envelope 41a filled with plastics pellets 41b. Consequently, prior to use the skull cap is non-rigid, and may be manually moulded to the shape of a patient's head.

The outlet ports 44 communicate, via a connector 50 with both an evacuator pump 51 and associated cock 52 and the inlet 43 via a reservoir 53, a heat exchanger 54, a liquid pump 55 and a connector 56. The heat exchanger 54 lies within an evaporator 57 which is part of a refrigerator 60, the refrigerator also comprising a compressor, 61, a condensor 62 and associated fan 63, and an expansion valve 64.

6

With the skull cap fitted over the head of a patient and connected as shown in figure 3, when the evacuator 51 is operated to bring the cap down to its working pressure the bead bag array clamps the cap to the head. Then with the evacuator 51 switched off and the pump 55 switched on, liquid drawn from the reservoir 53 and cooled in the heat exchanger 54 is passed via the connector 56 the inlet 43 and the diffuser 45 into the sac 40. It passes through the bead bags 41, which form an insulative reticulated structure to the heat pipe skull cap, and permeates the wick 42 to cool the head. Vapour given off by this passes via the diffusers 45, the outlets 44 and the connector 50 to condense in the reservoir 50. After use, release of the vacuum to the cap allows it to go floppy once again and be removed from the head.

CLAIMS

1. Apparatus for cooling a scalp and comprising

a heat pipe having a conditioning heat exchanger portion constructed for embracing a scalp,

a reservoir heat exchanger portion, and

a communicating duct portion connecting the conditioning and the reservoir heat exchanger portions.

2. Apparatus as claimed in claim 1 and formed integral in flexible sheet heat pipe form, the heat pipe having a reticulated structure including wicking and void continua, an impermeable plastics film envelope surrounding the structure, and means by which the heat pipe may be outgassed and evacuated and liquid introduced thereinto.

3. Apparatus as claimed in claim 1 and wherein the conditioning portion has a deeply dentate edge so that it can be formed to embrace a scalp of any shape.

4. Apparatus as claimed in claim 1 and wherein the reservoir heat exchanger has foldable flaps so that it can be formed into a container or a container lining.

5. Apparatus as claimed in claim 1 and incorporating a scalp cap and ties for maintaining the conditioning portion in contact with a scalp.

6. Apparatus as claimed in claim 1 and incorporating a scalp cap comprising a bag of pellets the bag fabric being substantially impermeable and the interior connectable to evacuating means, whereby the bag can be formed over the conditioner portion on a user's head, and evacuated to retain the conditioner portion in close contact with the head.

7    Apparatus as claimed in claim 1 and wherein the heat pipe comprises

a substantially impermeable sac for embracing the scalp,

at least one bag of pellets within the sac and at an outer part thereof and arrayed so as to be substantially coextensive therewith, and

a wicking pad within the sac and at an inner part thereof and substantially coextensive therewith.

8    Apparatus as claimed in claim 7 and wherein said at least one bag of pellets is permeable.

9    Apparatus as claimed in claim 7 and connected to an evacuator.

10    Apparatus as claimed in claim 7 and connected to a liquid pump.

11    Apparatus as claimed in claim 7 and wherein the reservoir heat exchanger portion comprises the evaporator of refrigeration apparatus.

12    Apparatus as claimed in claim 1 and wherein the communicating duct portion is sheathed in thermally insulative material.

13    Apparatus as claimed in claim 1 having an associated evacuating pump.

14    Apparatus as claimed in claim 1 having a scalp cap, the scalp cap comprising a bag of pellets, the bag fabric being substantially impermeable and its interior connected to an evaluating pump together with that of the heat pipe, whereby the bag can be formed over the conditioner portion on a user's head, and evacuated to retain the conditioner portion in close contact with the head.

15    The process of treatment of a hirsute skin and comprising covering the said skin with the conditioning portion of a heat pipe, and placing the reservoir portion of the heat pipe in contact with a low temperature source, the heat pipe being in accordance with the first aspect of the present invention claim 1 and charged with a non-aqueous working liquid which has been outgassed by evacuation to $10^{-4}$ -$10^{-2}$ Torr.

Fig.1.

10  12  11  23
22  21  20  22  21

Fig.2.

31
10
32
12
30

Fig. 3.